# EUROPEAN PATENT APPLICATION

(11) **EP 2 545 936 A2**
(43) Date of publication of application: **16.01.2013**
(21) Application number: 12165151.7
(22) Date of filing: 31.05.2007
(51) Int. Cl.: A61K 39/112, A61K 39/00, C07K 14/245, C07K 16/12

(54) **Methods, compositions, and kits for treating Shiga toxin associated conditions**

(30) Priority: 31.05.2006 US 809464 P
(62) Divisional of application: 07795522.7
(71) Applicant: Thallion Pharmaceuticals Inc., Montreal, QC H4S 2C8 (CA); The Henry M. Jackson Foundation for the Advancement of Military Medicine, Inc., Rockville, MD 20852 (US)
(72) Inventor: Riviere, Marc, Saint Lambert, Québec J4P 3B1 (CA); Mehran, Mariam, Blainville, Québec J7C 4Y3 (CA); Roberson, Claire, Plantation, FL 33317 (US); O'Brien, Alison, Bethesda, MD 20817 (US); Melton-Celsa, Angela, Sterling, VA 20165 (US); Sam-Pan, Janique, Laval, Québec H7K 3P9 (CA)
(74) Representative: Bird Goën & Co

(57) **Abstract**

The invention features methods, compositions, and kits for treating a subject having a Shiga toxin associated disease with chimeric anti-Shiga Toxin (cαStx1) and anti-Shiga Toxin 2 (cαStx2) antibodies

## Description

### BACKGROUND OF THE INVENTION

In general, this invention relates to the field of treating and preventing Shiga toxin associated diseases.

In the United States, Shiga toxin (Stx)-producing *Escherichia coli* (STEC) account for about 110,000 infections per year. Enterohemorrhagic E. *coli* (EHEC), most notably the serotype O157:H7, is a subset of STEC that is noted for producing Stx mediated disease. A possible complication from an infection with a Stx-producing organism is the hemolytic uremic syndrome (HUS), which is characterized by hemolytic anemia, thrombic thrombocytopenia, and renal failure. There is approximately a 5-10% fatality rate for those with HUS and survivors may have lasting kidney damage. Currently there are no FDA approved therapies or vaccines to combat or prevent illness from a Stx-mediated disease, but several promising options for the future include: a humanized monoclonal antibody that binds to and neutralizes Stx2 and a chimeric StxA2/StxB1 toxoid that elicits a neutralizing response and provides protection against a lethal challenge of Stx1 or Stx2 or Stx1 and Stx2.

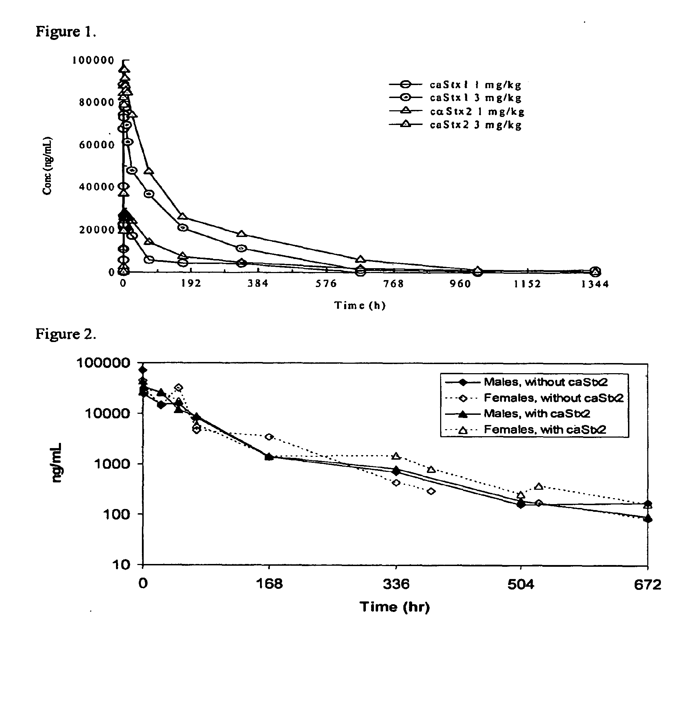

There are essentially two main types of Stxs: Stx/Stx1 and Stx2. Stx is produced from *Shigella dysenteriae* type 1, while Stx1 and Stx2 are produced from *Escherichia coli.* Stx and Stx1 are virtually identical, with only one amino acid difference in the A subunit. The mature A and B subunits of Stx1 and Stx2 have 68 and 73% similarity, respectively. Despite the amino acid sequence differences, the crystal structures of Stx and Stx2 are remarkably similar (Figure 1). These toxins can be differentiated by polyclonal antisera: polyclonal antisera raised against Stx1 does not neutralize Stx2 and vice-versa. Variants of Stx1 and Stx2 exist and include Stx1c, Stx1d, Stx2c, Stx2d, Stx2d-activatable (Stx2-act.), Stx2e, and Stx2f.

Shiga toxins are complex holotoxins with an AB₅ structure. The active domain (A), contains an *N*-glycosidase that depurinates the 28S rRNA of the 60S ribosomal subunit, which stops proteins synthesis and eventually leads to cell death. The A subunit is ~ 32 kDa and is proteolytically cleaved by trypsin or furin into a ~ 28 kDa A₁ subunit and a ~ 5 kDa A₂ peptide which are connected through a single disulphide bond. The A₁ subunit contains the active domain, and the A₂ peptide non-covalently tethers the active domain to the binding domain. The binding domain (B) consists of five identical ~ 7.7 kDa monomers that form a pentamer through which the C-terminus of the A₂ peptide traverses. Each of the B subunit monomers has two cysteine residues that form a disulphide bond within each monomer (Figure 2). The B pentamer binds the eukaryotic receptor globotriaosyl ceramide (Gb₃) (or Gb₄ as is the case for Stx2e).

Despite this knowledge about the results of exposure to these toxins, currently there is no known cure or vaccine for HUS. The use of antibiotics may exacerbate the situation by increasing toxin release from bacteria. Thus, there is a need for a compound to prevent or to treat the complications of EHEC produced by Shiga toxin. Such a compound could be used to treat infected subjects and decrease the systemic effects of toxin on the CNS, blood, and kidneys. In addition, if the toxin could be neutralized, antibiotics could be safely given to kill the bacteria in the GI tract. Such a compound could also be used to prevent infectious complications, by treating exposed or high risk individuals before they acquire EHEC infection. Such individuals would include children in day care or the elderly in nursing homes, where a case of EHEC diarrhea has been identified. These individuals are at increased risk to develop EHEC, often with severe complications, and spread of EHEC in these environments is not unusual.

### SUMMARY OF THE INVENTION

We have discovered that chimeric anti-Stx1 and chimeric anti-Stx2, when administered at 1 mg/kg or 3 mg/kg, are effective in treating Shiga toxin-associated diseases.

In one aspect, the invention features a method for the treatment of a Shiga toxin associated condition in a subject (e.g., a human) by administering an effective amount of chimeric anti-Stx1 and chimeric anti-Stx2 antibodies to the subject. Each of the chimeric antibodies is administered at 1 mg/kg or 3 mg/kg (for example by intravenous infusion over a period of 15, 30, 45, 60, 90, 120 minutes, or more. In a preferred embodiment, the antibodies are administered by intravenous infusion for between 30 minutes and 1 hour). In this aspect, the chimeric anti-Stx1 antibody includes a human IgG1-kappa immunoglobulin constant region, an immunoglobulin heavy chain variable region including the amino acid sequence set forth in SEQ ID NO: 1, and an immunoglobulin light chain variable region including the amino acid sequence set forth in SEQ ID NO:2. The chimeric anti-Stx2 antibody includes a human IgG1-kappa immunoglobulin constant region, an immunoglobulin heavy chain variable region including the amino acid sequence set forth in SEQ ID NO: 3, and an immunoglobulin light chain variable region including the amino acid sequence set forth in SEQ ID NO: 4.

In another aspect, the invention features an article of manufacture. This article of manufacture includes chimeric anti-Stx1 and chimeric anti-Stx2 antibodies and a label. The label indicates that the chimeric anti-Stx1 and chimeric anti-Stx2 antibodies are for treating a Shiga-toxin associated disease (e.g., in a human less than 18 years of age, less than 6 months old, and between 6 months and 3 years old)and are to be administered at a dosage of 1 mg/kg or 3 mg/kg each. In this aspect, the chimeric anti-Stx1 antibody includes a human immunoglobulin constant region IgG1-kappa, an immunoglobulin heavy chain variable region including the amino acid sequence set forth in SEQ ID NO:1, and an immunoglobulin light chain variable region including the amino acid sequence set forth in SEQ ID NO:2. The chimeric anti-Stx2 antibody includes a human immunoglobulin constant region IgGI-kappa, an immunoglobulin heavy chain variable region including the amino acid sequence set forth in SEQ ID NO: 3, and an immunoglobulin light chain variable region including the amino acid sequence set forth in SEQ ID NO: 4.

In yet another aspect, the invention features a kit. This kit includes chimeric anti-Stx1 and chimeric anti-Stx2 antibodies, instructions, and a label. The instructions are for administering the chimeric anti-Stx1 and chimeric anti-Stx2 antibodies at a dosage of 1 mg/kg or 3 mg/kg. In this aspect the label indicates that the chimeric anti-Stx1 and chimeric anti-Stx2 antibodies are for treating a Shiga-toxin associated disease (e.g., in a human less than 18 years of age, less than 6 months old, and between 6 months and 3 years old). Further, the chimeric anti-Stx1 antibody includes a human immunoglobulin constant region IgG1-kappa, an immunoglobulin heavy chain variable region including the amino acid sequence set forth in SEQ ID NO: 1, and an immunoglobulin light chain variable region including the amino acid sequence set forth in SEQ ID NO:2. The chimeric anti-Stx2 antibody includes a human immunoglobulin constant region IgG1-kappa, an immunoglobulin heavy chain variable region including the amino acid sequence set forth in SEQ ID NO: 3, and an immunoglobulin light chain variable region including the amino acid sequence set forth in SEQ ID NO: 4.

In any of the foregoing aspects, the chimeric anti-Stx1 and chimeric anti-Stx2 antibodies can be co-administered. Further, the subject being administered chimeric anti-Stx1 and chimeric anti-Stx2 antibodies can be less than 18 years old (e.g., less than 6 months old or between 6 months and 3 year old).

By "chimeric anti-Stx1" or "cαStx1" is meant a humanized antibody that specifically binds to Stx1 and includes an IgG1 kappa human immunoglobulin constant region and the murine 13C4 (ATCC Accession No. CRL 1794) variable region. This antibody is described in U.S. Patent Application Publication No. 20030170248, which is hereby incorporated by reference in its entirety.

By "chimeric anti-Stx2" or "caStx2" is meant a humanized antibody that specifically binds to Stx2 and includes an IgG1 kappa human immunoglobulin constant region and the murine 11E10 (ATCC Accession No. CRL 1987) variable region. This antibody is described in U.S. Patent Application Publication No. 20030170248, which is hereby incorporated by reference in its entirety.

By the terms "specifically binds to" is meant an antibody that binds to a protein (e.g., Stx1 or Stx2) with a K_{d} value of between 100 nM-1 pM. Antibody affinities may be determined using any of the assays known in the art including, but not limited to, surface plasmon resonance based assay, enzyme-linked immunoabsorbent assay (ELISA), and competition assays (e.g. RIA's).

By "co-administered" is meant introduction of the two antibodies simultaneously. In one embodiment, each antibody is introduced at the appropriate dosage into single container (e.g., a container containing a buffer or solution (e.g., a saline solution)). This mixture is then administered by intravenous infusion to the subject.

By "Shiga toxin associated disease" is meant any disease resulting from a pathogen expressing a Shiga toxin. The term "Shiga toxin associated disease" is meant to include hemolytic uremia syndrome, shigellosis, and diseases resulting from Shiga toxin-producing *Escherichia coli* and *S. dysenteriae* infection.

The term "treatment" refers to both therapeutic treatment and prophylactic or preventative measures. Those in need of treatment include those already with the condition as well as those in which the disorder is to be prevented.

The term "intravenous infusion"" refers to introduction of a drug into the vein of a subject over a period of time of 15, 30, 45, 60, 90, 120 minutes, or more. In a preferred embodiment, the antibodies are administered by intravenous infusion for between 30 minutes and 1 hour.

### BRIEF DESCRIPTION OF THE DRAWINGS

Figure 1 is a graph showing the concentration of cαStx1 and cαStx2 as a function of time after intravenous administration to healthy adult volunteers.
Figure 2 is a graph showing the serum concentration of cαStx1 with and without co-administration of cαStx2 as a function of time.
Figure 3 is a graph showing the serum concentration of cαStx2 with and without co-administration of cαStx1 as a function of time.
Figure 4 is a diagram showing the study population and database.
Figure 5 is a series of graphs showing the number of days of hospitalization as a function of score on the STEC Disease Severity Scale for the indicated patients. The STME "bloody diarrhea" was correlated with the outcome "days of hospitalization" (error bars are standard errors of the means).

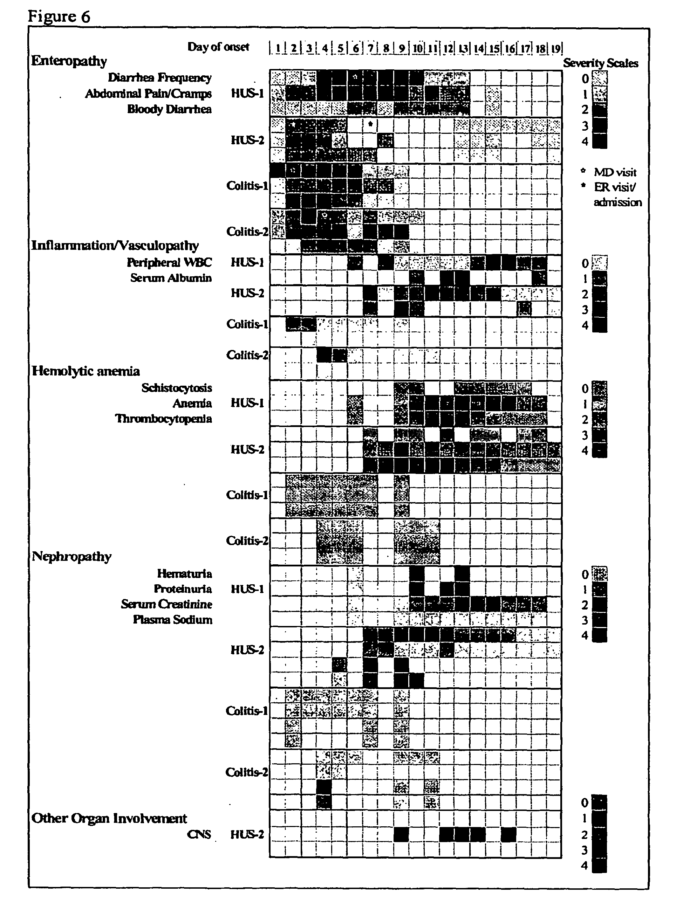

Figure 6 is a disease expression array showing four children with evolving STEC O157:H7 infection, two with incipient HUS (HUS-1, -2) and two with bloody diarrhea only (Colitis-1, -2), separated by bold lines. Each row represents a distinct clinical or laboratory item and its evolution over time, in the same order for each of the four patients. Columns indicate consecutive days, starting with Day 1 (=disease onset) for each patient. Severity of symptoms or laboratory abnormalities are visualized by different intensities of shading such that the degree of darkening corresponds to the score (0-4) in the proposed STEC Disease Severity and Progression scale (see Table 17). Empty fields represent days with missing information

### DETAILED DESCRIPTION OF THE INVENTION

In general the invention features methods, compositions, and kits for treating Shiga toxin associated diseases in a subject with chimeric anti-Shiga Toxin 1 (cαStx1) and chimeric anti-Shiga Toxin 2 (cαStx2) antibodies as defined herein.

### I. INDICATIONS

The compounds and methods of the invention are useful for treating subjects having, or at risk of developing a Shiga toxin associated disease. Such subject include subjects infected with *S. dysenteriae* or EHEC (Enterohemorrhagic *E. coli).* Such subjects would also include children in day care or the elderly in nursing homes. In one example, the subject is in a day care or in a nursing home where a case of EHEC diarrhea has been detected. In this example, the subject may or may not have developed the disease. Shiga toxin associated diseases include those resulting from infection with Shiga toxin producing *S. dysenteriae* or EHEC, most notably the serotype O157:H7 These infections often result in hemolytic uremic syndrome (HUS), which is characterized by hemolytic anemia, thrombotic thrombocytopenia, and renal failure.

### II. CHIMERIC αSTX1 AND αSTX2 ANTIBODIES

The invention features methods and compositions that include chimeric αStx1 and chimeric αStx2 antibodies for the treatment of Shiga toxin associated diseases. These antibodies are set forth in U.S. Patent Application Serial Nos. 09/215,163 and 11/471,420, each of which is incorporated by reference in its entirety. cαStx1 and cαStx2 are chimeric monoclonal IgG1 antibodies that bind to Shiga toxin 1 (Stx1) and Shiga toxin 2 (Stx2) respectively. cαStx1 recognizes the B subunit of Stx1 and cαStx2 recognizes the A subunit of Stx2.

The chimeric anti-Stx 1 antibody is a humanized antibody that specifically binds to Stx1 and includes an IgG1 kappa human immunoglobulin constant region and the murine 13C4 (ATCC Accession No. CRL 1794) variable region. In one embodiment, the murine heavy chain variable region includes the amino acid sequence set forth in SEQ ID NO: 1 and the murine light chain variable region includes the amino acid sequence set forth in SEQ ID NO:2. This antibody is described in U.S. Patent Application Publication No. 20030170248, which is hereby incorporated by reference in its entirety.

The chimeric anti-Stx2 antibody is a humanized antibody that specifically binds to Stx2 and includes an IgG1 kappa human immunoglobulin constant region and the murine 11E10 (ATCC Accession No. CRL 1987) variable region. In one embodiment, the murine heavy chain variable region includes the amino acid sequence set forth in SEQ ID NO:3 and the murine light chain variable region includes the amino acid sequence set forth in SEQ ID NO:4. This antibody is described in U.S. Patent Application Publication No. 20030170248, which is hereby incorporated by reference in its entirety.

### III. PHARMACEUTICAL FORMULATIONS

Therapeutic formulations of the antibodies used in accordance with the present invention can include an antibody having the desired degree of purity with optional pharmaceutically acceptable carriers, excipients or stabilizers (Remington: The Science and Practice of Pharmacy 21 st edition, University of the Sciences in Philadelphia Ed. 2005), in the form of lyophilized formulations or aqueous solutions. Acceptable carriers excipients, or stabilizers for intravenous administration are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; met al complexes (e.g. Zn-protein complexes); and/or non-ionic surfactants such as TWEEN™, PLURONICS™, or polyethylene glycol (PEG).

The formulation herein may also contain more than one active compound as necessary for the particular indication being treated, preferably those with complementary activities that do not adversely affect each other. For example, it may be desirable to further provide antibodies that binds Stx1 or Stx2 (e.g. an antibody which binds a different epitope on Stx1 or Stx2) in the one formulation.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington: The Science and Practice of Pharmacy 21st edition, University of the Sciences in Philadelphia Ed. 2005.

The formulations to be used for in vivo administration must be sterile. This is readily accomplished by filtration through sterile filtration membranes.

Sustained-release preparations may be prepared. Suitable examples of sustained-release preparations include semipermeable matrices of solid hydrophobic polymers containing the antibody, which matrices are in the form of shaped articles, e.g. films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37° C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

Formulations for oral use include tablets containing the active ingredient(s) in a mixture with non-toxic pharmaceutically acceptable excipients. These excipients may be, for example, inert diluents or fillers (e.g., sucrose, sorbitol, sugar, mannitol, microcrystalline cellulose, starches including potato starch, calcium carbonate, sodium chloride, lactose, calcium phosphate, calcium sulfate, or sodium phosphate); granulating and disintegrating agents (e.g., cellulose derivatives including microcrystalline cellulose, starches including potato starch, croscarmellose sodium, alginates, or alginic acid); binding agents (e.g., sucrose, glucose, sorbitol, acacia, alginic acid, sodium alginate, gelatin, starch, pregelatinized starch, microcrystalline cellulose, magnesium aluminum silicate, carboxymethylcellulose sodium, methylcellulose, hydroxypropyl methylcellulose, ethylcellulose, polyvinylpyrrolidone, or polyethylene glycol); and lubricating agents, glidants, and antiadhesives (e.g., magnesium stearate, zinc stearate, stearic acid, silicas, hydrogenated vegetable oils, or talc). Other pharmaceutically acceptable excipients can be colorants, flavoring agents, plasticizers, humectants, buffering agents, and the like.

The tablets may be uncoated or they may be coated by known techniques, optionally to delay disintegration and absorption in the gastrointestinal tract and thereby providing a sustained action over a longer period. The coating may be adapted to release the active drug substance in a predetermined pattern (e.g., in order to achieve a controlled release formulation) or it may be adapted not to release the active drug substance until after passage of the stomach (enteric coating). The coating may be a sugar coating, a film coating (e.g., based on hydroxypropyl methylcellulose, methylcellulose, methyl hydroxyethylcellulose, hydroxypropylcellulose, carboxymethylcellulose, acrylate copolymers, polyethylene glycols and/or polyvinylpyrrolidone), or an enteric coating (e.g., based on methacrylic acid copolymer, cellulose acetate phthalate, hydroxypropyl methylcellulose phthalate, hydroxypropyl methylcellulose acetate succinate, polyvinyl acetate phthalate, shellac, and/or ethylcellulose). Furthermore, a time delay material such as, e.g., glyceryl monostearate or glyceryl distearate may be employed.

The solid tablet compositions may include a coating adapted to protect the composition from unwanted chemical changes, (e.g., chemical degradation prior to the release of the active drug substance).

### IV. TREATMENT WITH cαSTX1 AND cαSTX2 ANTIBODIES

It is contemplated that, according to the present invention, cαstx1 and cαStx2 antibodies may be used to treat various Shiga toxin associated diseases. The antibodies of the invention are administered to a subject, (e.g., a human patient), in accord with known methods, such as intravenous administration as a bolus or by continuous infusion over a period of time, by intramuscular, intraperitoneal, intracerobrospinal, subcutaneous, intra-articular, intrasynovial, intrathecal, oral, rectal, topical, or inhalation routes. Intravenous administration of the antibody is preferred. In addition, the antibody or antagonist may suitably be administered by pulse infusion, e.g., with declining doses of the antibody.

For the prevention or treatment of disease, the appropriate dosage of cαStx1 and cαStx2 antibodies will depend on the type of disease to be treated, as defined above, the severity and course of the Shiga toxin associated disease, whether the antibody is administered for preventive or therapeutic purposes, previous therapy, the patient's clinical history and response to the antibody, and the discretion of the attending physician. The antibody is suitably administered to the patient at one time or over a series of treatments.

According to the invention, dosage regimens may include a dose of 1 mg/kg, or more preferably 3 mg/kg delivered by intravenous or subcutaneous infusion for each of the cαStx1 and cαStx2 antibodies. These two antibodies may administered as a single formulation or separate formulations. Where the antibody is well-tolerated by the patient, the time of infusion may be reduced.

This initial dose may be followed by subsequent follow-up doses by intravenous infusion, intravenous bolus injection, subcutaneous infusion, solid oral doses, or subcutaneous bolus injection.

### V. ARTICLES OF MANUFACTURE

In another embodiment of the invention, an article of manufacture containing materials useful for the treatment of the disorders described above is provided. The article of manufacture comprises a container, a label and a package insert. Suitable containers include, for example, bottles, vials, syringes, etc. The containers may be formed from a variety of materials such as glass or plastic. The container holds a composition which is effective for treating the condition and may have a sterile access port (for example, the container may be an intravenous solution bag or a vial having a stopper pierceable by a hypodermic injection needle). At least one active agent in the composition is either the cαStx1 or cαStx2 antibody or both. The label on, or associated with, the container indicates that the composition is used for treating the condition of choice. The article of manufacture may further comprise a second container comprising a pharmaceutically-acceptable buffer, such as phosphate-buffered saline, Ringer's solution and dextrose solution. It may further include other materials desirable from a commercial and user standpoint, including other buffers, diluents, filters, needles, and syringes. In addition, the article of manufacture may comprise a package inserts with instructions for use.

Furthermore, kits of the invention may include one, or both cαStx1 and cαStx2, preferably formulated for intravenous administration. Such kits may further include instructions for administering the antibodies at either 1 mg/kg or 3 mg/kg to a patient having or at risk of developing a Shiga toxin associated disease.

### VI. EXAMPLES

### Example 1

### Safety and Tolerability of Chimeric Anti-Shiga toxin 1 (cαStx1) and Shiga toxin 2 (cαStx2) in Healthy Adults

### Introduction

Currently there is no causal treatment for Shiga toxin producing bacterial (STPB) infection and its complications. Chimeric monoclonal antibodies against Shiga toxin 1 and Shiga toxin 2 (designated cαStx1 and cαStx2) have been developed to address this unmet medical need. Since human STPB can produce either or both Shiga toxins, antibodies against both toxins are needed to maximize chances for clinical success. In preclinical studies, neither of the two monoclonal antibodies was found to be associated with any systemic toxicity in two species.

The safety, tolerability and pharmacokinetics of the anti-toxins cαStx1 and cαStx2 in healthy adults from two Phase I studies are presented below.

### Objective

The primary objective of two clinical Phase I studies, conducted in healthy male and female adults, was to evaluate the safety and tolerability of chimeric monoclonal antibodies cαStx1 and cαStx2 following a single intravenous dose of the antibodies administered individually or concomitantly.

The secondary objectives of the studies were to:
i) evaluate the pharmacokinetics of cαStx1 and cαStx2 and
ii) assess the development of human anti-chimeric antibodies (HACA).

### Methods

Each study was a Phase I, single site, open label, non-randomized study. The primary study eligibility criteria are listed in Table 1. All study participants were healthy male and female volunteers.

**Table 1.**

| Primary Study Eligibility Criteria |
|---|
| **Inclusion criteria:** |
| -Male or female age 18 to 55 years inclusive |
| -BMI ≥19.0 and <24.9 kg/m2 |

| **Exclusion criteria:** |
|---|
| -Chronic condition requiring daily prescription medication |
| -History or presence of diabetes, cancer, heart disease, autoimmune disease, mental illness, CNS disorder, seizure, respiratory disorder or renal disorder |
| -Abnormal laboratory tests judged clinically significant |
| -Positive screening tests for hepatitis B, C or HIV |
| -ECG abnormalities (clinically significant) or vital sign abnormalities (systolic blood pressure lower than 90 or over 140 mmHg, diastolic blood pressure lower than 50 or over 90 mmHg, or heart rate less than 50 or over 100 bpm) at screening |

The chimeric antibodies were administered as a single dose at the concentrations listed in Table 2. The study treatment was diluted in 100 mL of saline and infused at 100 mL/hr for 1 hour through a dedicated intravenous line.

**Table 2.**

| Study Treatment | | |
|---|---|---|
| **Number of Participants** | **Dose cαstx1 (mg/kg)** | **Dose cαStx2 (mg/kg)** |
| 4 | 1 | - |
| 4 | 3 | - |
| 4 | - | 1 |
| 4 | - | 3 |
| 5 | 1 | 1 |
| 5 | 3 | 3 |

The main study visit assessments are listed in Table 3.

**Table 3.**

| Study Visit Assessments | | | | | | | | | | |
|---|---|---|---|---|---|---|---|---|---|---|
| Assessment | Screening Day -30 to Day -1 | Day-1 | Day 1 | Day 2 | Day 4 | Day 8 | Day 15 | Day 29 | Day 43 | Day 57 or ET |
| Physical Exam | x | x | | x | x | x | x | x | x | x |
| ECG | x | | x* | x | | x | x | | | x |
| Safety Laboratory | x | x | | x | x | x | x | x | | x |
| Study Medication Administration | | | x | | | | | | | |
| PK | | | x* | x | x | x | x | x | x | x |
| HACA | | | x | | | | | x | x | x |

| | | | | | | | | | | |
|---|---|---|---|---|---|---|---|---|---|---|
| ET = Early Terminadon * = multiple assessments HACA = Human Anti-Chimeric Antibody | | | | | | | | | | |

PK serum samples were collected at the following intervals: predose, 0.25 h, 0.5 h, 1 hour (end of study medication infusion), 1.25 h, 1.5 h, 2 h, 3 h, 4 h, 5 h, 7 h, 9 h, 12 h, 24 h (Day 2), 72 h (Day 4), 168 h (Day 8), 336 h (Day 15), 672 h (Day 29), 1032 h (Day 43), and 1344 h (Day 57) after start of study medication infusion. PK serum samples were analyzed to determine the cαStx1 and cαStx2 concentrations using validated ELISA methods. PK parameters (Cmax, Tmax, AUC (0 - t), AUC(0-inf), λz, t½, CL and Vz) were calculated by standard non-compartmental methods for each antibody.

Serum samples were analyzed for anti-cαStx1 and anti-cαStx2 antibodies using validated ELISA methods.

All analyses were based on the Safety Population, which was comprised of any participant who enrolled and who received any amount of study medication.

### Results

Twenty-five (25) of the twenty-six (26) participants completed the study to Day 57. One participant in the combined 1 mg/kg cαStx1 and 1 mg/kg cαStx2 group was lost to follow-up after the Day 2 visit.

No serious adverse events were observed and 4 of the 26 (15%) participants did not experience any adverse event. The most frequent adverse events reported are presented in Table 4 and were mild to moderate in severity.

**Table 4.**

| **Most Frequent Adverse Events** | | | | | | | |
|---|---|---|---|---|---|---|---|
| **AE** | **No. Participants with AE I No. Participants per Dose Group** | | | | | | **Total** |
| | **cαstx1 1 mg/kg** | **cαStx1 3 mg/kg** | **cαStx2 1 mglkg** | **cαStx2 3 mg/kg** | **cαStx1/cαStx2 1/1 mg/kg** | **cαStx1/cαStx2 3/3 mg/kg** | |
| Abd Cramps | 0/4 | 0/4 | 0/4 | 0/4 | 1/5 | 1/5 | 2/26 |
| Back Pain | 1/4 | 1/4 | 1/4 | 0/4 | 0/5 | 0/5 | 3/26 |
| Bloating | 0/4 | 0/4 | 0/4 | 0/4 | 0/5 | 3/5 | 3/26 |
| Cold Symptoms | 0/4 | 1/4 | 0/4 | 0/4 | 1/5 | 0/5 | 2/26 |
| Cough | 2/4 | 0/4 | 0/4 | 1/4 | 0/5 | 0/5 | 3/26 |
| Diarrhea | 0/4 | 1/4 | 0/4 | 0/4 | 0/5 | 2/5 | 3/26 |
| Fever | 1/4 | 1/4 | 0/4 | 0/4 | 0/5 | 0/5 | 2/26 |
| Headache | 2/4 | 0/4 | 0/4 | 0/4 | 2/5 | 1/5 | 5/26 |
| Nausea | 0/4 | 1/4 | 0/4 | 1/4 | 0/5 | 1/5 | 3/26 |
| Leucocyturia | 0/4 | 0/4 | 0/4 | 1/4 | 0/5 | 2/5 | 3/26 |
| Erythema at Blood Draw Site | 0/4 | 0/4 | 0/4 | 0/4 | 2/5 | 0/5 | 2/26 |
| Erythema at Infusion Site | 0/4 | 0/4 | 0/4 | 0/4 | 1/5 | 1/5 | 2/26 |
| Sleepiness | 0/4 | 0/4 | 0/4 | 0/4 | 4/5 | 1/5 | 5/26 |
| Sore Throat | 1/4 | 0/4 | 1/4 | 1/4 | 0/4 | 0/4 | 3/26 |

There was no apparent relationship between the dose of antibodies and the number of participants who experienced an adverse event and, in general, abnormal laboratory parameters were not clinical significant. There was no trend between the dose of cαStx1 or cαStx2 and the occurrence of abnormal laboratory values and except the fever reported in Table 4, there were no clinically significant changes in body weight, vital signs or electrocardiogram measurements (heart rate, PR, QRS, and QT/QTc).

Mean serum concentrations of cαStx1 and cαStx2 in study participants administered the antibodies individually are shown in Figure 1.

The pharmacokinetic results for each study participant in the individual antibody administration study are shown in Table 5. The determination of the pharmacokinetic results for the combined antibody administration study is in progress.

**Table 5.**

| **Pharmacokinetic Parameters for cαStx1 and cαStx2 Administered Individually** | | | | |
|---|---|---|---|---|
| **Participant No.** | **Dose Group** | **Cmax (ng/mL)** | **AUC_{Inf}** | **t½ (hours)** |
| 001 | 1 mg/kg cαStx1 | 23155.29 | 1976510.68 | 97.701 |
| 002 | 1 mg/kg cαStx1 | 28636.93 | N/A | N/A |
| 003 | 1 mg/kg cαStx1 | 25562.79 | 2156130.90 | 85.588 |
| 004 | 1 mg/kg cαStx1 | 21644.56 | 2293940.55 | 112.164 |
| 005 | 3 mg/kg cαStx1 | 86707.01 | 10651193.50 | 155.691 |
| 006 | 3 mg/kg cαStx1 | 85035.24 | 18089272.33 | 184.153 |
| 007 | 3 mg/kg cαStx1 | 112543.33 | N/A | N/A |
| 008 | 3 mg/kg cαStx1 | 86116.34 | 8114842.87 | 282.396 |
| 009 | 1 mg/kg cαStx2 | 25086.41 | 7244993.38 | 314.921 |
| 010 | 1 mg/kg cαStx2 | 29603.41 | 7214392.22 | 430.969 |
| 011 | 1 mg/kg cαStx2 | 28666.08 | 2477386.00 | 60.832 |
| 012 | 1 mg/kg cαStx2 | 33610.09 | 6131600.07 | 418.828 |
| 013 | 3 mg/kg cαStx2 | 77715.58 | 18405696.29 | 251.760 |
| 014 | 3 mg/kg cαStx2 | 104325.34 | 13362783.90 | 188.628 |
| 015 | 3 mg/kg cαStx2 | 104839.75 | 23071589.07 | 219.575 |
| 016 | 3 mg/kg cαStx2 | 115883.56 | 17131825.36 | 211.623 |

Three (3) out of 26 participants exhibited an induction of human anti-chimeric antibody (HACA) at concentrations close to the detection limit as per Table 6.

**Table 6.**

| HACA Response | | | |
|---|---|---|---|
| Participant No. | Dose Group | Sampling Day | Antibody Concentration (ng/mL) |
| 016 | 3 mg/kg cαStx2 | Day 57 | 56.94 |
| 007 | 3 mg/kg cαStx1 and 3 mg/kg cαStx2 | Day 29 | 8.40 |
| | | Day 57 | 18.60 |
| 008 | 3 mg/kg cαStx1 and 3 mg/kg cαStx2 | Day 29 | 8.19 |

The standard curve range was 7.81-750 ng/mL. None of the samples tested in either study yielded results above the lower limit of quantitation for binding against cαStx1.

### Conclusions

No study participants had their study medication infusion interrupted or discontinued due to an adverse event (AE) or abnormal finding. No participants dropped out of the studies due to an AE and none experienced a SAE and there were no consistent changes in body weight, vital signs or electrocardiogram measurements between the individual or combined antibody dose groups of cαStx1 and cαStx2.

There is an expected proportionality of an approximately 3-fold increase in the Cmax and AUC between the 2 doses for each antibody. At 3 mg/kg the half-life of cαStx1 and cαStx2 is approximately 9 days. Human anti-chimeric antibody (HACA) response was minimal.

At the termination of each study a safety committee, which consisted of an independent medical monitor and the Principal Investigator, reviewed the laboratory results, ECG tracings and AEs of all study participants and concluded that cαStx1 and cαStx2 administered individually or concomitantly up to 3 mg/kg per antibody was safe and well tolerated. Overall, anti-toxins cαStx1 and cαStx2 administered intravenously individually or concomitantly at a dose up to 3 mg/kg per antibody were determined to be safe and well tolerated in healthy adult males and females.

### Example 2

### Toxicology and Immunogenicity of cαStx1 and cαStx2 in Mice and Marmosets

### Introduction

Shiga toxin producing Escherichia coli (STEC) are zoonotic pathogens that cause potentially fatal and often epidemic and food or waterborne illness with a clinical spectrum that includes diarrhea, hemorrhagic colitis and hemolytic uremic syndrome (HUS)(Karmali MA, et al. J Infect Dis, 2003: 188 (1 Dec) 1724-1729). STEC produces two distinct Shiga toxin types, Shiga toxin 1 and Shiga toxin 2 (Karmali MA, et al. J Infect Dis, 2004: 189 (1 Feb) 355-359). Currently there is no specific treatment against STEC infection but two specific anti-toxins are in development to treat infections due to Shiga toxin producing bacteria (STPB).

The toxicology and immunogenicity of the anti-toxins tested in two animal species are presented in this example. The mouse was tested because it is a universally used model for evaluating the toxicity of various classes of chemicals and for which there is a large historical database. Additional toxicology was conducted in the non-rodent marmoset primate model.

### Objective

The objective of two non-clinical studies conducted in mice and marmosets was to evaluate the potential acute toxicity and immunogenicity of each chimeric monoclonal antibody, cαStx1 and/or cαStx2, following a single or repeat dose of the antibodies administered individually or concomitantly to healthy animals.

### Methods

In the mouse study, one hundred sixty-seven male and female (167) CD1 mice experimentally naïve and approximately 7 weeks in age were included. All mice had body weights that fell within ± 20% of the mean body weight for each sex. Animals were randomized by sex into treatment groups. Each animal was implanted with a microchip bearing a unique identification number. Mice were housed individually in suspended, stainless steel, wire-mesh type cages. Temperature was maintained at 18 to 26°C and humidity at 30 to 70%. A 12 hour dark cycle was controlled automatically to provide a cycle of 12 hours of light and 12 hours of dark, unless other light/dark schedules were required by the experimental design. Mice were fed meal Lab Diet® Certified Rodent Diet #5002, PMI Nutrition International, Inc. and water ad libitum.

Dosing solutions were prepared on the day of dose administration. Mice were given a single bolus injection via the tail vein of cαStx1, cαStx2 or both cαStx1 and cαStx2 on Day 1 or Day 1 and Day 8 of the study per Table 7. Dosing solution samples were collected and analyzed for protein concentration by bicinchoninic acid (BCA). Mice serum samples collected on Day 29 were analyzed for anti-cαStx1 and anti-caStx2 antibodies and isotyping using validated ELISA methods.

Cageside observation and a detailed clinical examination of the skin, fur, eyes, ears, nose, oral cavity, thorax, abdomen, external genitalia, limbs, feet and respiratory, circulatory and nervous systems was performed daily.

Body weights for all mice were measured and recorded the day after arrival, just prior to randomization (Day -1) and weekly during the study.

Blood and urine samples were taken for clinical pathology and serum antibody evaluation as defined in Table 7.

Macroscopic, microscopic and organ weight evaluations were conducted post-sacrifice according to the times in Table 7. The mice were examined macroscopically for external abnormalities including masses. The abdominal, thoracic and cranial cavities were examined for abnormalities and the organs removed, examined and placed in fixative. Organ weights for the adrenal gland, brain, heart, kidney, liver, lung, ovary, pituitary, testis, thymus, thyroid (with parathyroid) and uterus with cervix were recorded for all animals at the scheduled necropsies and appropriate organ weight ratios were calculated (relative to body and brain weights). Microscopic examinations were performed on the fixed sections of brain, heart, injection site, kidney, large intestine (cecum, colon, rectum), liver, lung, lymph node (inguinal), small intestine (duodenum, ileum, jejunum), spleen, thymus and gross lesions per Table 7.

**Table 7.**

| **Mouse Study Design** | | | | | | | | | | | | | |
|---|---|---|---|---|---|---|---|---|---|---|---|---|---|
| **Grp No.** | **Number of mice** | | **Dose mg/kg of each antibody (IV bolus)** | **Time of Dose** | **Clinical radiology collection times (Hematology, serum biochemistry serum antibody collection times biochemistry, and evaluation)** | | | **Necropsy / Organ collection times** | | | **Microscopic Pathology** | | |
| | **Projected** | | | | | **Actual** | | | **Actual** | | | **Actual** | |
| | **M** | **F** | | | | **M** | **F** | | **M** | **F** | | **M** | **F** |
| 1a | 10 | 10 | 0(Fb) | Day 1 | Day 8* | 10 | 10 | Day 8 | 10 | 10 | AR | 1 | 2 |
| 1b | 10 | 10 | 0(Fb) | Day 1.8 | Day 15* | 10 | 10 | Day 15 | 10 | 10 | Day 15 | 10 | 10 |
| 1c | 10 | 10 | 0(Fb) | Day 1, 8 | Day 29** | 10 | 10 | Day 29 | 10 | 10 | Day 29 | 10 | 10 |
| 2a | 10 | 10 | 30 | Day 1 | Day 8* | 10 | 10 | Day 8 | 10 | 10 | AR | - | 2 |
| 2b | 10 | 10 | 30 | Day 1 | Day 29** | 10 | 10 | Day 29 | 10 | 10 | AR | 1 | - |
| 3a | 10 | 10 | 30 x 2 | Day 1, 8 | Day 15* | 10 | 3* | Day 15 | 10 | 3 | Day 15 | 10 | 3 |
| 3b | 10 | 10 | 30 x 2 | Day 1, 8 | Day 29** | 10 | 4* | Day 29 | 10 | 4 | Day 29 | 10 | 10 |
| 4 | 10 | 10 | cαStx1@30 | Day 1 | Day 29** | 10 | 10 | Day 29 | 10 | 10 | AR | - | - |
| 5 | 10 | 10 | cαStx2@ 30 | Day 1 | Day 29** | 10 | 10 | Day 29 | 10 | 10 | AR | - | - |

| | | | | | | | | | | | | | |
|---|---|---|---|---|---|---|---|---|---|---|---|---|---|
| **Fb** = formulation buffer, Group I dosed 2 x 6 mL/Kg of vehicle except for animals sacrificed on Day 8 (1 dose). * : 5/sex/group used for hematology analysis and 5/sex/group used for clinical chemistry. ** : 5/sex/group used for hematology & serum antibody analysis and 5/sex/group used for clinical chemistry. **AR:** Microscopic evaluation was performed as required based on microscopic pathology evaluations of Group 3. a, b: Due to lack of product only 7 of the 20 females dosed on Day I received the second dose on Day 8 (all animals survived). a: 2 of the 3 females dosed for Group 3a were used for hematology analysis and I was used for clinical chemistry. b: 2 of the 4 females dosed for Group 3b were used for hematology & serum antibody anal & 2 were used for clinical chem. Statistical comparisons were conducted between the mouse controls in group 1 and the treated mice in groups 2, 3, 4 and 5. | | | | | | | | | | | | | |

In the marmoset study twenty-two male and female marmosets **(44** in total) experimentally naive and approximately **1** to 5 years old (Callithrixjacchus) were used in the study after 9 weeks acclimation. Males placed on the study had body weights from 299 to 372 g and females 313 to 435 g. Animals were randomly allocated to dosing groups based on stratified body weight. Each animal was implanted with a microchip bearing a unique identification number. Marmosets were housed individually by sex in a climate controlled room. Temperature was maintained at 24 to 28°C and relative humidity at 30 to 70%. A 12 hour light/dark cycle was maintained. Each animal was offered a variety of food which was prepared fresh each day and given twice a day according to a meal plan. Animals were also given food rewards immediately after each handling/manipulation. Tap water was provided ad libitum via an automatic water system or bottles except during urine collection.

Dosing solutions were prepared on the day of dose administration. Male and female marmosets were administered cαStx1, cαStx2 or both cαStx1 and cαStx2 on Day 1 or Day 1 and Day 8 of the study per Table 8. The test item was administered by an ambulatory infusion pump over 30 minutes through an indwelling catheter in the leg or arm vein. Dosing solution samples were collected and analyzed for protein concentration by bicinchoninic acid (BCA).

All marmosets were observed cageside twice daily throughout the duration of the study for morbidity, mortality, injury, behavior, appearance and feces. Body weights for all marmosets were measured and recorded at least once predose, just prior to randomization (Day -1) and weekly during the study.

Indirect ophthalmoscopy was performed on all animals once during the predose phase and before necropsy.

Heart rate, RR, PR, QRS, QT and QTc intervals were measured by electrocardiography once during the predose phase and directly after the end of the 30 minute infusion on Day 1 or Day 8.

Blood samples for clinical pathology (hematology and chemistry) and serum antibody evaluation were taken from the femoral vein/artery for designated marmosets on Days 8, 15 or 29 according to the sampling schedule in Table 8. Samples for urinalysis over a 16 hour period were also collected as per Table 8. Marmoset serum samples were analyzed for anti-cαStx1 and anti-cαStx2 antibodies and isotyping using validated ELISA methods.

Macroscopic, microscopic and organ weight evaluations were conducted postmortem according to the times in Table 8. A full macroscopic examination was performed. Organ weights for all the same organs as listed in the mice study along with eyes and optic nerve, epididymides, prostate, seminal vesicles and spleen were recorded for all surviving animals at the scheduled necropsies and appropriate organ weight ratios were calculated (relative to body and brain weights). Microscopic examinations were performed on the same fixed sections as listed in the mice study along with that of the eyes and optic nerve, skin and stomach per Table 8.

**Table 8.**

| Marmoset Study Design | | | | | | | | | | | | | | | |
|---|---|---|---|---|---|---|---|---|---|---|---|---|---|---|---|
| **Grp No.** | **Number of Marmosets** | | **Antibody (30 minutes IV infusion)** | | **Dose mg/kg. each Mab** | **Time of Dose** | **Antibody & ClinicalPatholgy Blood & urine collection times** | | | **Necropsy/Organ Collection Times** | | | **Microscopic pathology** | | |
| | **Projected** | | | | | | | **Actual** | | | **Actual** | | | **Actual** | |
| | **M** | **F** | **αSt X1** | **αStx2** | | | | **M** | **F** | | **M** | **F** | | **M** | **F** |
| 1a | 2 | 2 | - | - | 0 (Fb) | Day 1 | Day 8 | 2 | 2 | Day 8 | 2 | 2 | AR | * | |
| 1b | 2 | 2 | - | - | 2x0 (Fb) | Day1,8 | Day 15 | 2 | 2 | Day 15 | 2 | 2 | Yes | 2 | 2 |
| Ac | 2 | 2 | - | - | 2x0 (Fb) | Day 1,8 | Day 29 | 2 | 2 | Day 29 | 2 | 2 | Yes | 2 | 2 |
| 2a | 2 | 2 | Yes | Yes | 30 | Day 1 | Day 28 | 2 | 2 | Day 28 | 2 | 2 | Ar | * | * |
| 2b | 2 | 2 | Yes | Yes | 30 | Day 1 | Day 29 | 2 | 2 | Day 29 | 2 | 2 | Ar | * | * |
| 3a | 2 | 2 | Yes | Yes | 2x30 | Day1,8 | Day 15 | 2 | 2 | Day 15 | 2 | 2 | yes | 2 | 2 |
| 3b | 2 | 2 | Yes | Yes | 2x30 | Day1,8 | Day 29 | 2 | 2 | Day 29 | 2 | 2 | Yes | 2 | 2 |
| 4a | 2 | 2 | Yes | - | 30 | Day 1 | Day 8 | 2 | 2 | Day 8 | 2 | 2 | AR | * | * |
| 4b | 2 | 2 | Yes | - | 30 | Day 1 | Day 29 | 2 | 2 | Day 29 | 2 | 2 | AR | * | * |
| 5a | 2 | 2 | - | Yes | 30 | Day 1 | Day 8 | 2 | 2 | Day 8 | 2 | 2 | AR | * | * |
| 5b | 2 | 2 | - | Yes | 30 | Day 1 | Day 29 | 2 | 2 | Day 29 | 2 | 2 | AR | * | * |

| | | | | | | | | | | | | | | | |
|---|---|---|---|---|---|---|---|---|---|---|---|---|---|---|---|
| **Fb** = formulation buffer, **Mab=** Monoclonal antibody **AR:** Microscopic evaluation was performed As Required based on microscopic pathology evaluations of Group 3. **YES:** performed after end of in -life - All animals survived. * : Microscopic pathology was not required based on pathology evaluations of Group 3. | | | | | | | | | | | | | | | |

Data from marmosets that were administered the monoclonal antibodies were compared with the control data. No statistical analyses were performed.

### Results

All dosing solution concentrations were within acceptance values for slope, intercept and R2 and reproducibility of sample values were within 6%. All animals from both studies survived the in-life phase of the study until their scheduled termination. Test article related changes for the endpoints observed are presented in Table 9 for the mice and marmosets respectively.

**Table 9.**

| Mice and Marmosets Test Article Related Effects | | |
|---|---|---|
| **Endpoints observed** | **Test article-related changes** | |
| | **Mice** | **Marmosets** |
| **Cage side observations:** | | |
| Body weights: | None | None |
| Food consumption: | None | None |
| Feces: | N/A | None |
| Indirect ophthalmoscopy: | N/A | None |
| Electrocardiography: | N/A | None |
| Detailed clinical examinations: | None | None |

| **Clinical pathology:** | | |
|---|---|---|
| Hematology: | None | None |
| Clinical chemistry: | None | None |
| Urine analysis: | None | None |

| **Necropsy examinations:** | | |
|---|---|---|
| Macroscopic observations: | None | None |
| Organ weights: | None | None |
| Microscopic examinations: | None | None |
| | | |

| **Comparisons:** | | |
|---|---|---|
| Controls versus treatment groups: | No test article-related changes | No test article-related changes |
| Gender difference: | No gender difference | No gender difference |

In the mouse study 2 animals exhibited a low mouse anti-human antibody (MAHA) response to cαStx2 on Day 29. None of the mice that received cαStx1 yielded results above the lower limit of quantitation (LLOQ) for binding against cαStx1. The concentrations and isotyping of the positive responses are presented in Table 10. Of the 44 marmoset samples tested, none were found to contain anti-therapeutic antibodies above the LLOQ.

**Table 10.**

| Mouse Serum Antibody Concentration Analysis for Binding Against anti-cαStx2 | | | | |
|---|---|---|---|---|
| **Sample ID** | **Gender** | **Dose** | **Antibody Conc.** | **Isotype** |
| 6067 | Male | 60/60 mg/kg (caStx1/caStx2) | 159.25ng/Ml | IgG1, IgG2b |
| 6180 | Female | 30mg/kg (caStx2) | 373.80ng/mL | IgG1, IgG2b |

### Conclusions

cαStx1 and cαStx2, administered intravenously individually or concomitantly, at a single or repeat dose of 30 mg/kg or 60 mg/kg per antibody respectively, were well tolerated and did not show any consistent adverse treatment-related effects in male and female mice or marmosets. The anti-therapeutic response was minimal and only limited to mice.

In conclusion, the monoclonal antibodies against Shiga toxins 1 and 2 were tested in two species at doses up to 20 times above the intended clinical dose and were not associated with any systemic toxicity.

### Example 3

### Pharmacokinetics of cαStx1 and cαStx2 in Mice

### Introduction

Shiga toxins 1 and 2 are the virulence factors that are responsible for the complications that come from infection by Shiga toxin producing bacteria (STPB) (Gavin PJ, et al. J Clin Microbiology, Apr 2004, p.1652-1656). Shiga toxin producing Escherichia coli (STEC) strains represent the most important recently emerged group of food-borne pathogens (Blanco JE, et al. J Clin Microbiology, Jan 2004, p. 311-319). STEC causes a potentially fatal foodbome illness whose clinical spectrum includes asymptomatic carriage, nonspecific diarrhea, bloody diarrhea, hemorrhagic colitis and hemolytic uremic syndrome (HUS) (Karmali MA, Molecular Biotechnology, Vol. 26, 2004, p. 117-122; Klein EJ, et al. J Pediatr, Aug 2002, Vol 141, No. 2, p. 172-177). Two specific monoclonal antibodies against Shiga toxin 1 and 2 have been developed to treat STPB infection.

cαStx1 and cαStx2 are chimeric monoclonal IgG1 antibodies that bind with Shiga toxin 1 (Stx1) and Shiga toxin 2 (Stx2) respectively. cαStx1 recognizes the B subunit of Stx1 and cαStx2 recognizes the A subunit of Stx2.

The pharmacokinetics of these specific antitoxins in mice are presented in this example.

### Objective

The objective of this study was to evaluate the pharmacokinetic profile of each monoclonal antibody following a single dose of antibodies cαStx1 and/or cαStx2 administered individually or concomitantly to healthy CD-1 mice.

### Methods

One hundred thirty male and female CD1 mice were used in the study after 1 week of acclimation. All mice placed on the study had body weights that fell within ± 20% of the mean body weight for each sex. Animals were randomized by sex into treatment groups using a standard block randomization procedure. Each animal was implanted with a microchip bearing a unique identification number. Mice were housed in suspended, stainless steel, wire-mesh type cages. Temperature was maintained at 64 to 79°F and humidity at 30 to 70%. A 12 hour dark cycle was provided via an automatic timer for approximately 12 hours per day. Mice were fed meal Lab Diet® Certified Rodent Diet #5002, PMI Nutrition International, Inc. and water ad libitum.

Dosing solutions were prepared on the day of dose administration. Male and female mice were given a single bolus injection via the tail vein of cαStx1, cαStx2 or both cαStx1 and cαStx2 on Day 1 of the study per Table 11. Blood samples were taken via the maxillary vein for the first designated time points and via cardiac puncture under anesthesia for the last or only designated time point according to the sampling schedule in Table 11.

**Table 11.**

| Study Design | | | | | | |
|---|---|---|---|---|---|---|
| **Table 1. Study Design** | | | | | | |
| **Group** | **Test Article** | **# Male** | **# Female** | **Dose Level (mg/kg)** | **Dose Volume (mL/kg)** | **Sample Time Post Dose** |
| i | FB | 5 | 5 | 0 | 1.0 | 1 hr |
| ii | cαStx1 | 5 | 5 | 3 | 1.0 | 10 min |
| iii | αStx1 | 5 | 5 | 3 | 1.0 | 1 hr. 72 hrs, Day 7 |
| iv | cαStx1 | 5 | 5 | 3 | 1.0 | 24 hrs, Day 14, Day 28* |
| v | cαStx1 | 5 | 5 | 3 | 1.0 | 48hrs, Day 21^{b} |
| vi | cαStx2 | 5 | 5 | 3 | 1.0 | 10 min |
| vii | cαStx2 | 5 | 5 | 3 | 1.0 | 1 hr, 72 hrs, Day 7 |
| viii | cαStx2 | 5 | 5 | 3 | 1.0 | 24 hrs, Day 14, Day 28* |
| ix | cαStx2 | 5 | 5 | 3 | 1.0 | 48hrs, Day 21^{b} |
| x | cαStx1 / cαStx2 | 5 | 5 | 3/3 | 1.0 | 10 min |
| xi | cαStx1 / cαStx2 | 5 | 5 | 3/3 | 1.0 | 1 hr, 72 hrs, Day 7 |
| xii | cαStx1 / cαStx2 | 5 | 5 | 3/3 | 1.0 | 24 hrs, Day 14, Day 28* |
| xiii | cαStx1 / cαStx2 | 5 | 5 | 3/3 | 1.0 | 48hrs. Day 21^{b} |

| | | | | | | |
|---|---|---|---|---|---|---|
| FB = formulation buffer • Female animals (3/5 in Group iv, 3/5 in Group viii and 1/5 in Group xii had samples drawn on Day 22). • Female animals (4/5 in Group v. 3/5 in Group ix and 1/5 in Group xiii had samples drawn on Day 16). | | | | | | |

Samples of dosing formulations at each concentration were collected pre and post dose administration on Day 1 and analyzed for protein concentration by bicinchoninic acid (BCA). Serum samples were analyzed to determine the cαStx1 and cαStx2 concentrations using validated ELISA methods. Nominal sample collection times were used in the pharmacokinetic data analysis. Serum concentrations of cαStx1 and cαStx2 were used to construct semi-logarithmic serum concentration versus time curves. Pharmacokinetic parameters were determined by non-compartmental methods. Values below the limit of quantitation were treated as 0.

Observations for mortality, morbidity, injury and availability of food and water were conducted twice daily for all animals. Mice body weights were measured on Day -1, Day 1, and weekly thereafter. Necropsy examinations were performed on animals that died on study.

### Results

No basal diet or water contaminants likely to interfere with the results of this study were reported. There was no test article related mortality or adverse clinical observations. All dosing solution concentrations were within acceptance values for slope, intercept and R2 and reproducibility of sample values were within 6%.

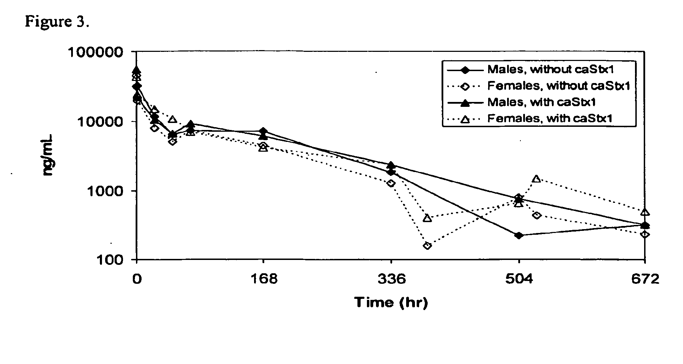

The minimum concentration of cαStx1 and cαStx2 in the serum samples that could be accurately determined by ELISA was 130 ng/mL and 150 ng/mL respectively. Serum concentrations of cαStx1 and cαStx2 in male (22.5 - 37.0 g) and female (21.6 - 29.9 g) mice administered alone or in combination are shown in Figures 2 and 3.

The pharmacokinetic parameters of area under the serum concentration - time curve from time 0 to infinity (AUCO-∞), the terminal half-life (t½), serum clearance (CL) and volume of distribution at steady state (Vss) are presented in Table 12.

**Table 12.**

| Pharmacokinetic Parameters for cαStx1 and cαStx2 Administered Alone or in Combination | | | | | | | | |
|---|---|---|---|---|---|---|---|---|
| | **AUC** **(hr•ng/mL)** | | **CL** **(mL/kg/min)** | | **t_{1/2}** **(hr)** | | **V** **(mL/kg)** | |
| | M | F | M | F | M | F | M | F |
| cαStx1 alone | 1.760.000 | 2.310.000 | 0.0284 | 0.0216 | 89.2 | 159 | 156 | 109 |
| cαStx2 alone | 2.570.000 | 1.880.000 | 0.0195 | 0.0266 | 99.0 | 113 | 159 | 240 |
| cαStx1 (co-administered with cαStx2) | 2.230.000 | 2.260.000 | 0.0225 | 0.0222 | 90.6 | 98.1 | 103 | 129 |
| cαStx2 (co-administered with cαStx1) | 2.640.000 | 2.460.000 | 0.0189 | 0.0203 | 117 | 116 | 193 | 211 |

### Conclusions

Serum concentrations of cαStx1 and cαStx2 were similar in male and female mice whether administered alone or in combination. There were no consistent differences between males and females in the other pharmacokinetic parameters calculated. CL was low for both cαStx1 and cαStx2 which is consistent with the long half-lives and expected for monoclonal antibodies. Vss was also low for both cαStx1 and cαStx2 which is indicative that the antibodies are retained within the blood volume. Combined administration of cαStx1 and cαStx2 did not alter AUCO-oo, t½, CL or Vss.

PK results are consistent with the structure of the anti-Shiga toxin monoclonal antibodies and with the known absence of cross-reactivity with human or animal tissues.

### Example 4

### Renal Involvement in Children with STEC Colitis

### Introduction

The definition of hemolytic uremic syndrome (HUS) includes hemolytic anemia, thrombocytopenia and acute nephropathy/renal failure. Up to 25% of children who survive STEC associated (enteropathic) hemolytic uremic syndrome (eHUS) develop long-term renal abnormalities. As Garg et al pointed out, the prognosis of acute, self-limited Escherichia coli O157:H7 gastroenteritis has never been previously studied (Garg et al., Kidney Int. 2006; 70: 807-812). A recent cohort-control study, initiated after the Walkerton outbreak due to contaminated drinking water, which affected about 2000 people, demonstrated slightly increased rates of hypertension and reduced kidney function in affected adults four years after outbreak-associated STEC (plus C. jejuni) diarrhea (Garg et al., CMAJ. 2005;173: 261-268; Garg et al., Kidney Int. 2005; 67: 1476-1482). These studies do not include laboratory analyses at the time of the STEC infection. The current analysis was undertaken to address the question whether children with STEC diarrhea without HUS demonstrate disease-associated renal abnormalities.

### Objectives

To determine whether children with acute STEC colitis demonstrate urinary/renal abnormalities.

### Design

Retrospective chart review and data analysis. Data are from a single, tertiary care university-affiliated pediatric hospital with an active emergency medicine department. The hospital serves >1 million total population.

### Methods

Patients with STEC isolates between 1992 and 2006 were identified from the hospital microbiology laboratory records. Available clinical and demographic information was extracted from medical charts (paper and electronic, if available) using a standardised questionnaire and entered into the Montreal Children's STEC Disease database, which now comprises 186 children with culture-proven STEC 0157 infection.

Data were analysed with respect to final diagnosis (STEC diarrhea, HUS), demographic information

For the purposes of this study, the diagnostic criteria for indicated diseases are as follows: haematuria, urine RBC (red blood cells) > 5 per HPF (high power field) or dipstick analysis greater than "trace"; proteinuria, urine protein ≥0.3 g/l (30 mg/dl) or urine protein:creatinine ratio (U p/c) > 0.2 g/g; pyuria, urine WBC (white blood cells) >5 per HPF or positive dipstick analysis; and azotaemia, serum creatinine level exceeding 1.5 x reference limit for age and gender. Day of first STEC infection-related symptoms (generally diarrhea or abdominal cramps), referred to as DDO (Day of disease onset)

### Results

Matching urinalyses, plasma creatinine and hematological profiles were available from 103/186 patients with E. coli 0157 isolates (mostly O157:H7). Twenty-two patients developed HUS. Hematuria, defined as positive dipstick analysis or >5 RBC per high power field (HPF), was noted in 14 patients with non-HUS colitis (16.4%) on day 6 of onset (median; range 1-10 days); nine patients (10.6%) had proteinuria ≥0.3 g/l on day 6 (4-15). Conversely, 3/6 patients with matching laboratory studies prior to apparent HUS presented with hematuria on day 5 (3-6), and 2/6 with proteinuria on days 3, corresponding to two days before HUS. Age-adjusted plasma creatinine levels were normal in all patients except those with manifest HUS. These data are set forth in Tables 13-16.

**Table 13.**

| **Diagnosis** | **N** | **Urinalysis¹** | **Results** | |
|---|---|---|---|---|
| | | | Haematuria | Proteinuria |
| STEC colitis/diarrhea only | 163 | 85 | 14 (16.4%) | 9 (10.6%) |
| HUS | 22 | 18 | 18 (100%) | 17 (94.4%) |
| Total | 186 | 103 | 32 (31.1%) | 25 (24.3%) |

**Table 14**

| **Diagnosis** | **N (positive test result)** | **Urinalysis¹** | **First Positive Test Result (Day of Disease Onset)¹** | |
|---|---|---|---|---|
| | | | Haematuria | Proteinuria |
| STEC colitis/diarrhea only | 14 | Mean (SD) | 6.2 ± 2.6 | 7.1 ± 3.9 |
| | | Median (range) | 6 (1-10) | 5.5 (4-15) |
| HUS | 18 | Mean (SD) | 7.1 ± 3.4 (n = 18) | 7.3 ± 3.0 (n = 17) |
| | | Median (range) | 8 (2-14) | 8 (2-14) |

| | | | | |
|---|---|---|---|---|
| ¹ Within 15 days of disease onset | | | | |

**Table 15**

| **Scoring of urinalysis results in patient with STEC infection¹** | | | |
|---|---|---|---|
| | | **Most Abnormal Test Result** | |
| | | **Haematuria** | **Proteinuria** |
| Non-HUS | Mean (SD) | 2.1± 0.9 (n = 14) | 1.8 ± 1.0 (n = 9) |
| | Median (range) | 2 (0-3) | 1 (1-3) |
| HUS | Mean (SD) | 2.9 ± 0.5 (n = 18) | 2.9 ± 0.25 (n = 16) |
| | Median (range) | 3 (1-3) | 3 (2-3) |

| | | | |
|---|---|---|---|
| ¹ Scores as defined in Table 16 | | | |

**Table 16**

| STEC Disease Severity and Progression (SDSP) Scale | | | | | | |
|---|---|---|---|---|---|---|
| **Nephropathy** | | | | | | |
| **Category** | **Test item** | **0** | **1** | **2** | **3** | **4** |
| **Renal function** | Serum creatinine¹ | < 1.5 x upper normal | 1.5 to 2x upper normal | >2 to 4x upper normal | >4x upper normal | Dialysis |
| **Haematuria** | RBC [per HPF] | ? 5 | >5-10 | >10-30 | >30 | Gross haematuria |
| | Dipstick analysis | Negative or trace | Small | Moderate | Large | |
| **Proteinuria** | Dipstick [g/l] (mg/dl) | <0.3 (30) | 0.3 - <1 (30-100) | 1 - <3 (100-<300) | ? 3.0 (?300) | - |
| | U prot/creat [g/g] | <0.2 | 0.2 - <1 | 1 - <3 | ?3.0 | - |
| **Pyuria** | WBC [per HPF] | ?6 | 6-10 | >10-50 | >50 | - |
| | Leukocyte esterase (dipstix) | Negative | Positive 1+ | Positive 2+ | Positive 3+ | - |

| | | | | | | |
|---|---|---|---|---|---|---|
| ¹ relative to age and gender | | | | | | |

### Conclusion and Discussion

16% of children with non-HUS STEC colitis were found to have proteinuria and/or haematuria, compared to 50% of pre-HUS patients ≥2 days before microangiopathic hemolytic anemia became evident (falling hemoglobin and/or platelets or increasing serum creatinine). These results indicate that STEC may induce HUS-independent subtle renal injury mirroring findings in follow-up studies of children with bona fide HUS. The clinical significance and pathomechanism of STEC-colitis associated urinary abnormalities, if any, is unclear. Similar arguments apply to patients with "incomplete" HUS lacking evidence of renal failure.

### Example 5

### A Quantitative Disease Scale for Shiga Toxin Producing Escherichia coli Infections

### Introduction

Infections by Shiga toxin (Stx) producing E. coli (STEC) can lead to severe complications and result in significant human morbidity and economic losses. Hemorrhagic colitis (HC), haemolysis, nephropathy and central nervous system involvement are thought to result from the absorption of in vivo produced Stx into the regional (colon) or systemic circulation. Evidence suggests that Stx-mediated injury to the endothelium and other tissue causes a disordered microvascular and inflammatory response.

A limited set of signs and symptoms has been linked to Stx-inflicted tissue injury based on clinical observation, animal experimentation and cell biology experiments. Some of these presumed Stx-mediated events (STMEs) precede the appearance of the enteropathic haemolytic uremic syndrome (eHUS): cramping abdominal pain, bloody diarrhoea or hemorrhagic colitis. Others are part of HUS, such as thrombocytopenia, hemolytic anemia and various degrees of renal injury, but have also been noted in patients not fulfilling the diagnostic criteria of HUS ("incomplete" HUS). Rare extraintestinal and extrarenal manifestations (cerebral ischemia/stroke, seizures, cardiomyopathy, diabetes mellitus) are also thought to be caused by Stx or Stx-induced effects. Stx (or STEC infection) imparts a distinct inflammatory response associated with the induction of IL-8, TNFα, acute phase reactants and vasoactive mediators (Tarr PI et al. 2005; Bitzan M et al. 1998), which may explain why elevated peripheral neutrophil counts and C-reactive protein correlate positively with increased HUS risk.

While the clinical diagnosis of haemolytic uraemic syndrome (HUS) and HC is straightforward, criteria to define the severity or progression of STMEs and other clinical signs, or to evaluate the effect of therapeutic or preventive measures are lacking.

### Objectives

To generate and test a quantitative disease severity scale reflecting STEC and Stx-induced microvascular and tissue injury and to apply the resultant disease scale in a retrospective analysis using a well-described cohort of children with culture-proven STEC infection.

### Methods

■ Literature search for descriptions of STEC infections and markers/predictors of (complicated) HUS
■ Selection and adaptation of relevant items of the Common Terminology Criteria for Adverse Events (CTCAE V.3.0), based on known manifestations of STEC infections including HUS and expert opinion.
■ Identification of all patients with available STEC isolates from medical microbiology laboratory records.
■ Review of patients' medical files (paper charts and electronic records) and extraction of pertinent demographic, laboratory and clinical information using a standardized questionnaire.
■ Construction of a dedicated STEC disease database.

### Results

### Patient population and Database

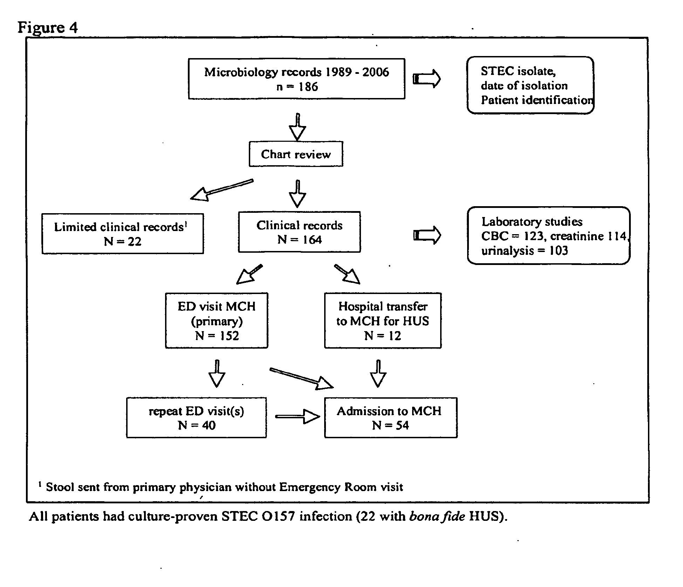

The presence and evolution of relevant clinical signs and symptoms, commonly available biological markers, and outcomes, if documented, of 186 patients with a wide spectrum of STEC disease manifestations, diagnosed between 1992 and 2006, were captured to create the comprehensive Montreal Children's STEC Disease (MCSD) database. Patient population and generation of the database are depicted in Figure 4.

### Creation of a Disease Severity Scale

A set of disease-related clinical and biological parameters was selected and assigned linear numerical values, in analogy to the Common Terminology Criteria for Adverse Events. Items were grouped into four pathological categories: enteropathy, inflammation and vasculopathy, microangiopathic hemolytic anemia and nephropathy (Table 17), plus extraintestinal and extrarenal organ involvement (not shown). The instrument is called "Shiga toxin/STEC Disease Severity and Progression" (SDSP) scale.

**Table 17.**

| STEC/Shiga toxin Severity and Progression Scale (abbreviated) | | | | | | | | | | |
|---|---|---|---|---|---|---|---|---|---|---|
| **Grade** | | **0** | **1** | | **2** | | **3** | | **4** | |
| **A Enteropathy** | | | | | | | | | | |
| Diarrhea (stool frequency) | | No diarrhea (baseline) | <5 | | 5 - <10 | | 10 - <15 | | ? 15 | |
| Abdominal pain or cramps Age-adjusted pain scale | | No pain (0) | Mild pain (1-3) | | Moderate pain (4-6) | | Severe pain (7-9) | | Unbearable pain (10) | |
| Bloody diarrhea (hemorrhage) | | No visible blood | Occasional streaks of blood | | Blood mixed with stool | | Frank blood (colorectal hemorrhage) | | Hemorrhage requiring intervention | |

| **Grade** | | **0** | **1** | | **2** | | **3** | | **4** | |
|---|---|---|---|---|---|---|---|---|---|---|
| **B Inflammation & Vasculopathy** | | | | | | | | | | |
| WBC [N/nl] < 2 yrs | | <15 | - | | 15 - <18 | | 18 - <22 | | ?22 | |
| 2-5.9 yrs | | <12 | 12 - <14 | | 14 - <18 | | 18 - <22 | | ?22 | |
| ? 6 yrs | | < 10.0 | 10- <14 | | 14 - <18 | | 18 - <22 | | ?22 | |
| Cerebrovascular ischemia | | None | - | | Migraine-type headache | | Transient ischemic attacks **(TIA)** | | Stroke, neurological deficit or blindness | |

| **Grade** | | **0** | **1** | | **2** | | **3** | | **4** | |
|---|---|---|---|---|---|---|---|---|---|---|
| **B Inflammation & Vasculopathy** | | | | | | | | | | |
| WBC [N/nl] < 2 yrs | | <15 | - | | 15 - <18 | | 18 - <22 | | ?22 | |
| 2-5.9 yrs | | <12 | 12 - <14 | | 14 - <18 | | 18 - <22 | | ?22 | |
| ? 6 yrs | | < 10.0 | 10- <14 | | 14 - <18 | | 18 - <22 | | ?22 | |
| **Cerebrovascular ischemia** | | None | - | | Migraine-type headache | | Transient ischemic attacks (TIA) | | Stroke, neurological deficit or blindness | |

| **Grade** | **0** | | | **1** | | **2** | | **3** | **4** | |
|---|---|---|---|---|---|---|---|---|---|---|
| **D Nephropathy** | | | | | | | | | | |
| Hematuria (RBC per HPF or dipstick) | None (<6) | | | Small (6-10) | | Moderate (>10-30) | | Large (>30) | | - |
| Proteinuria ([g/l or prot/creat] | <0.3 (<0.2 g/g) | | | 0.3 - <1 (0.2 - <1 g/g) | | 1 - <3 (1 -<3 g/g) | | ?3 (>3 g/g) | | - |
| Pyuria [WBC/HPF] | ?3 | | | 3-10 | | >10-50 | | >50 | | - |
| Serum creatinine (relative for age, gender) | Normal | | | ? 1.5 -2 x upper normal | | >2 -4x upper normal | | >4x upper normal | | Dialysis |
| Hyponatremia [µM] | ?135 | | | <134-131 | | <131-127 | | <127 | | Hyponatremic seizure |

### Validation of the STEC Disease Severity Scale

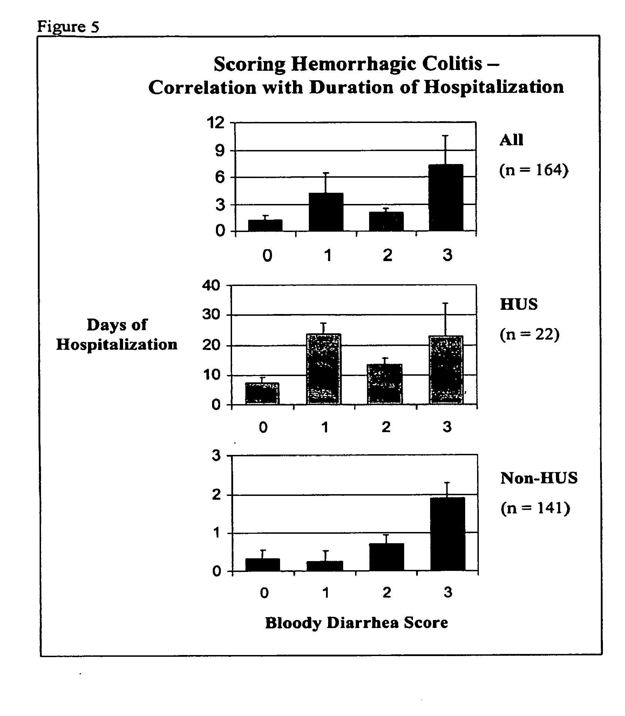

To validate the "Scale", scores for presumed STMEs and other clinical markers were compared with relevant outcome measures. An example is shown in Figure 5. The STME "bloody diarrhea" was correlated with the outcome "days of hospitalization". We demonstrate that a high score for bloody diarrhea translates into increased hospitalization. Not unexpectedly, however, this correlation is lost in patients with HUS due to secondary events dictating their hospital stay (compare the two lower panels in Figure 5).

### Utility of the STEC Disease Severity Scale

The "Scale" was conceived to "measure", in numeric, unified terms, the severity and evolution of STEC infections, e.g. in intervention studies. An additional, novel application is demonstrated in Figure 6. We used the scale to visually display the dynamics of the disease. We tentatively labeled this method "Disease Expression Array."

### Summary and Conclusions

We developed a new instrument, the "Shiga toxin/STEC Disease Severity and Progression" (SDSP) scale, as a tool to quantify STMEs and other clinical and laboratory abnormalities in patients with a wide spectrum of STEC infections.

### Example 6

### Epidemiological Evaluation of Shiga Toxin Producing Escherichia coli (STEC) Infections in the European Community

### Background

The majority of published reports on the epidemiology of STEC infections focus on outbreaks and the haemolytic uraemic syndrome (HUS). Publications providing global epidemiological figures, particularly of incident STEC infection rates in Europe, are scarce. Diagnostic challenges and reporting bias resulted in variable and distorted estimates of STEC infection and of extraintestinal complications hampering risk/benefit analyses for future preventive or therapeutic strategies.

### Objective

To establish the incidence of STEC infections in the European Community utilizing a variety of publicly available data sources and modelling strategies.

### Methods

We have estimated the prevalence of STEC infections in Europe based on the literature using three different approaches:
- Extrapolation of STEC infection incidence from STEC registries and other estimates reported as such in the literature.
- Extrapolation of the number of STEC infections based on literature assessment of HUS incidence, assuming that HUS represents 15% of the total STEC infections; a sensitivity analysis was also made using a 5% rate of HUS in STEC infections.
- Extrapolation of the number of STEC infections based on the literature reported incidence of childhood gastroenteritis assuming that a certain proportion of them should be STEC as reported by a few prospective papers.

### Results

The three different approaches lead to different estimates - not a surprising situation given the clinical context. No evaluation of the prevalence of the condition in the adult and elderly has been made given the absence of information in the literature

### Extrapolation of STEC infection incidence from STEC registries

### STEC in Finland

Study reported by Keskimaki in 1998 on the 1990-1997 period; in the first part of that period the prevalence of STEC was evaluated to be 0.013 cases per 100 000 per year; it was later realized that the majority of STEC infections are due to non-0157:H7 strains; the incidence was evaluated to 0.73 cases per 100 000 (Keskimäki et al., J Clin Microbiol 36, 3641-3646 (1998)).

### STEC infections in Spain

Blanco et al. published in 2004 a study on stool specimens of patients from the Lugo Hospital in Spain. STEC strains were detected in 2.5% of the 5,054 cases; O157:H7 represented 0.5% of cases and non-O157:H7 represented the balance (Blanco et al., J Clin Microbiol 42, 311-319 (2004)).

### STEC infections in Denmark

Ethelberg reported 425 patients with STEC infections in Denmark from January 1997 to May 2003 (Ethelberg et al., Emer Infect Dis 10, 842-847 (2004)). Most of the cases were sporadic.

### STEC infections in Netherlands

In 2004, Havelaar evaluated the median number of incident cases of STEC to be 1250 cases per year for the Netherlands (Havelaar et al., Epidemiol Infect 132, 467-484 (2004)). The estimate was derived from a case-control study which found 4 out of 798 faecal samples tested to be positive for STEC - a rate close to that reported in Spain.

### STEC infections in Ireland

Garvey and McKeown published in 2003 data derived from the National Disease Surveillance Centre (NDSC) for the 1999-2003 period (Garvey and McKeown, Epidemiology of Verotoxigenic E. coli 0157 in Ireland, 2003. NDSC (2003)). The surveillance was limited to O157:H7 (Table 18).

**Table 18.**

| **Crude Incidence Rate of O157:H7 infections in Ireland** | | |
|---|---|---|
| **Year** | **Numbers of cases (incl. non-residents)** | **Crude incidence rate (95% Cl) per 100,000 population** |
| 1999 | 51 | 1.4 (1.0-1.8) |
| 2000 | 37(42) | 0.9 (0.6-1.3) |
| 2001 | 50 (52) | 1.3 (0.9-1.6) |
| 2002 | 68 (70) | 1.7 (1.3-2.2) |
| 2003 | 82 (86) | 2.1 (1.6-2.6) |

### Extrapolation of the number of STEC infections based on HUS incidence

### HUS incidence in France

Incidence of HUS in 1998 was 0.7 cases per 100 000 children aged under 15 years (Haeghebaert et al., Eurosurv 5, 68-73 (2000)). Population under 15 years of age represents 11 121 100 persons over 59 630 100 or 18.65% of the French population (Eurostat 2004). Incidence of HUS would therefore be estimated to be 0.131/100 000 in the overall population.

### HUS incidence in Germany and Austria

A four-year prospective multicentre study across Germany and Austria was conducted between 1997 and 2000 (Gerber et al., J Infect Dis 186, 493-500 (2002)). 394 paediatric HUS reported translate into an annual incidence in Germany of 0.71 cases per 100 000 children <15 years (range 0.69-0.75); in Austria the number were respectively 0.51 cases for 100 000 children.

In Germany the population <16 years of age is 12 415 500, out of a total population of 82 536 700 or 15.04% (Eurostat 2004); the incidence of HUS in the general population would be 0.107/100 000.

In Austria the population <16 years of age is 1 335 200 out of a total population of 8 067 300 or 16.55%; the incidence of HUS would be 0.060/100 000.

### HUS incidence in the UK and Ireland

The average annual incidence (Lynn et al., Emerg Infect Dis 11, 590-596 (2005)) was 0.71/100 000 in children <16 years of age in the most recent cohort compared to 0.79 in a previous cohort. UK population <16 years of age is 11 126 200 out of a UK total population of 59 328 900 or 18.75%; the incidence of HUS being 0.71/100 000 in this population, the overall incidence rate would be 0.133/100 000.

### HUS incidence in Italy

Tozzi published in 2003 an incidence of HUS in Italian children <15 years of 0.28 per 100 000 over a 13 year surveillance period (Tozzi et al., Emer Infect Dis 9, 106-108 (2003)).

Data regarding HUS incidence in Europe are set forth in Table 19.

**Table 19.**

| HUS incidence per 100 000 in Europe | | | | | |
|---|---|---|---|---|---|
| | **Population < 15 years of age (000's)** | **Total population (000's)** | **% of population <15 years of age** | **Incidence in <16 years of age population per 100 000** | **incidence in the entire population per 100 000** |
| EU-25 | 75 415 | 453685 | 16.62% | 0.79 | 0.13132 |
| EU-15 | 82 886 | 379484 | 16.51% | 0.79 | 0.130456 |
| Eurozone | 48 915 | 305831 | 15.99% | 0.79 | 0.126354 |

HUS is a complication of approximately 15% of STEC infections, however the rate of HUS following STEC infection varies greatly in the different literature reports. To take this variation into account we have used a 5% rate of HUS occurrence to serve as a sensitivity analysis as displayed in Table 20 below.

**Table 20**

| Incidence of STEC infections per 100 000 in Europe - derived from HUS rates | | | | | |
|---|---|---|---|---|---|
| | **Total population (000's)** | **STEC incidence if 15% HUS rate (per 100,000)** | **STEC incidence if 5% HUS rate (per 100,000)** | **Total number of STEC cases if 15% HUS rate** | **Total number of STEC cases if 5% HUS rate** |
| EU-25 | 453 685 | 0.875466 | 2.626397 | 3 972 | 11 916 |
| EU-15 | 379484 | 0.86971 | 2.609129 | 3 300 | 9 901 |
| Eurozone | 305 831 | 0.842357 | 2.527072 | 2 576 | 7 729 |

### Extrapolation of the number of STEC infections based on the literature reported incidence of childhood gastroenteritis

The prospective assessment of community acquired gastroenteritis published by Frühwirth et al. in 2001 was our proxy reference for the incidence of gastroenteritis in Europe (Früwirth et al., Arch Dis Child 84, 393-397 (2001).). The study population at risk comprised 6969 children up to the age of 4 years followed-up in prospective, population based, multicentre study. The incidence of acute gastroenteritis was 4.67 per 100 children per year. We extrapolated that incidence to the <15 years old age group of the three different European populations defined by Eurostat to evaluate the maximum number of gastroenteritis that could be observed in the age group. Rate of STEC infections in community-acquired gastroenteritis was based on the Blanco 2004 report where the rate of STEC infections was 2.5% of the stools. These numbers are comparable to the 4 cases of STEC reported in a 798 stools series in Netherlands (Table 21).

**Table 21**

| Sensitivity analysis based on STEC rate in community acquired gastroenteritis | | | | | | | |
|---|---|---|---|---|---|---|---|
| | **Population <15 years of age (000's)** | **Total population (000's)** | **% of population 15 years of age** | **Gastroenteritis incidence per 100 000** | **Extrapolated incidence in the entire population per 100000** | **STEC incidence per 100 000 (if STEC rate is 2.5%)** | **Number of cases** |
| EU-25 | 75415 | 453685 | 16.62% | 4670 | 776.28 | 19.41 | 88,047 |
| EU-15 | 62666 | 379484 | 16.51% | 4670 | 771.18 | 19.28 | 73,163 |
| Eurozone | 48915 | 305831 | 15.99% | 4670 | 746.93 | 18.67 | 57,108 |

### Prevalence in Europe

STEC incidence range using the direct reports of STEC incidence in Europe or derived from the HUS incidence is 0.09 -0.26/10,000 cases.
The sensitivity analysis gives an incidence of 1.94/10 000 cases.
In conclusion a conservative estimate of Shiga toxin producing bacterial infection in the entire EU is 2 in 10 000.

### Prevalence in comparable jurisdictions

Epidemiological data reported in the USA (Mead et al., Emer Infect Dis 5, 607-625 (1999)). 110-220 cases of STEC infections was the annual incidence estimated by the CDC. The Population Reference Bureau estimated the US population to be 300 million in 2006. The annual number of cases reported to this population gives an incidence of 3.68/10 000 inhabitants.

In Australia, the incidence of HUS in children under 5 years (1.35 (95% CI 1.06 to 1.72) per 105) was lower than that reported in Canada (3.11 per 105 children <5 years) and the British Isles (3.3 per 105 children) (Elliott et al., Arch Dis Child 85, 125-131 (2001)).

In Canada, the annual rate of STEC infections was reported to be 1,21/10 000 cases (for the general population) in Alberta in the late 1980's (Waters et al., Clin Infect Dis. Nov;19(5):834-43 (1994)), which seems to have the highest rate of STEC infections in Canada. No other comprehensive epidemiological review was recently published.

All together the data from non-European developed countries is consistent with the European epidemiological data.

### Summary

The first approach, based on data for the years 1999-2003, yielded an incidence of STEC infections of 0.73-2.1/100,000 population. Using HUS frequencies (second approach), the incidence of STEC infections was calculated as 0.88 (for a HUS rate of 15%) to 2.63/100,000 population (HUS rate 5%). Using the third approach, a STEC infection rate of 7.8 to 19.4 per 100,000 population was calculated assuming that 1 - 2.5% of all diarrhea cases are due to STEC.

### Conclusion

Based on literature analysis, registry data and extrapolation from incidence figures for HUS and childhood diarrhoea, we propose that the demonstrated discrepancies in STEC infection rates are due to underreporting and that the maximal, true incidence in the EU-25 population is 19.4 per 100,000.

### OTHER EMBODIMENTS

Various modifications and variations of the described methods and compositions of the invention will be apparent to those skilled in the art without departing from the scope and spirit of the invention. Although the invention has been described in connection with specific desired embodiments, it should be understood that the invention as claimed should not be unduly limited to such specific embodiments. Indeed, various modifications of the described modes for carrying out the invention that are obvious to those skilled in the fields of medicine, immunology, pharmacology, endocrinology, or related fields are intended to be within the scope of the invention.

All patents, patent applications, and publications mentioned in this specification are herein incorporated by reference to the same extent as if each independent publication was specifically and individually incorporated by reference.

## Claims

1. A chimeric anti-Stx1 or a chimeric anti-Stx2 antibody for use in a method of treating a Shiga-toxin-associated condition in a subject, wherein said antibody is administered at a dosage of 1 mg/kg or 3 mg/kg of each antibody in a subject, **wherein said anti-Stx1 antibody comprises:**
a) a human IgG1-kappa immunoglobulin constant region;
b) an immunoglobulin heavy chain variable region comprising the amino acid sequence set forth in SEQ ID NO:1; and
c) an immunoglobulin light chain variable region comprising the amino acid sequence set forth in SEQ ID NO:2;
**and wherein said anti-Stx2 antibody comprises:**
d) a human IgG1-kappa immunoglobulin constant region;
e) an immunoglobulin heavy chain variable region comprising the amino acid sequence set forth in SEQ ID NO: 3; and
f) an immunoglobulin light chain variable region comprising the amino acid sequence set forth in SEQ ID NO: 4.

2. The antibody for use as defined in claim 1, wherein said method comprises use of both said anti-Stx1 antibody and said anti-Stx2 antibody.

3. The antibody for use as defined in claim 2, wherein each of said antibodies is administered as a dosage of 3 mg/kg.

4. The antibody for use as defined in any one of claims 1 to 3, wherein said antibody is formulated for intravenous administration, for intravenous infusion over at least 30 minutes, or for intravenous infusion over a period between 30 minutes and 1 hour.

5. The antibody for use as defined in any one of claims 1 to 4, wherein said subject is a human, a human less than 18 years old, a human between 6 months and 3 years old, or a human less than 6 months old.

6. An article of manufacture comprising the anti-Stx1 antibody or the anti-Stx2 antibody of claim 1 and a label, wherein said label indicates that said anti-Stx1 antibody or said anti-Stx2 antibody is for treating a Shiga-toxin-associated disease and is to be administered at a dosage of 1 mg/kg or 3 mg/kg.

7. The article of manufacture of claim 6, wherein said article of manufacture comprises both of said anti-Stx1 antibody and said anti-Stx2 antibody.

8. The article of manufacture of claim 6 or 7, wherein said label indicates that said anti-Stx1 antibody and/or anti-Stx2 antibody is to be administered at a dosage of 3 mg/kg.

9. The article of manufacture of any one of claims 6 to 8, wherein said article of manufacture comprises a composition comprising both said anti-Stx1 antibody and said anti-Stx2 antibody.

10. A kit comprising the anti-Stx1 antibody or the anti-Stx2 antibody of claim 1, instructions, and a label, wherein said instructions are for administering said anti-Stx1 antibody or said anti-Stx2 antibody at a dosage of 1 mg/kg or 3 mg/kg, wherein said label indicates that said anti-Stx1 antibody or said anti-Stx2 antibody is for treating a Shiga-toxin-associated disease.

11. The kit of claim 10, wherein said kit comprises both said anti-Stx1 antibody and said anti-Stx2 antibody and said instructions are for administering said anti-Stx1 antibody and said anti-Stx2 antibody at a dosage of 1 mg/kg or 3 mg/kg each.

12. The kit of claim 11, wherein said anti-Stx1 antibody and said anti-Stx2 antibody are provided in separate compositions.

13. The kit of claim any one of claims 10, 11, or 12, wherein said instructions are for administering said anti-Stx1 antibody and/or said anti-Stx2 antibody intravenously.

14. A pharmaceutical formulation comprising one or both of the chimeric anti-Stx1 antibody and the chimeric anti-Stx2 antibody of claim 1 for administration to a subject at a dosage of 1 mg/kg or 3 mg/kg.

15. The pharmaceutical formulation of claim 14, wherein said pharmaceutical formulation is a lyophilized formulation or is an aqueous solution.


SEQUENCE LISTING

<110>  Thallion Pharmaceuticals Incorporated et al.

<120>  Methods, Compositions, and Kits for Treating Shiga Toxin
       Associated Conditions

<130>  50111/115WO2

<150>  US 60/809,464
<151>  2006-05-31

<160>  4

<170>  PatentIn version 3.3

<210>  1
<211>  122
<212>  PRT
<213>  Shigella dysenteriae

<400>  1

Gln Val Gln Leu Gln Glu Ser Gly Ala Glu Leu Val Arg Ser Gly Ala
1               5                   10                  15

Ser Val Arg Met Ser Cys Asp Ala Ser Gly Tyr Thr Phe Thr Ser Tyr
            20                  25                  30

Asn Met His Trp Val Lys Gln Thr Pro Gly Gln Gly Leu Glu Trp Ile
        35                  40                  45

Gly Tyr Ile Tyr Pro Gly Asn Gly Gly Thr Asn Tyr Ile Gln Lys Phe
    50                  55                  60

Lys Gly Lys Ala Ile Leu Thr Ala Asp Thr Ser Ser Ser Thr Ala Tyr
65                  70                  75                  80

Met Gln Ile Ser Ser Leu Thr Ser Glu Asp Ser Ala Val Tyr Phe Cys
                85                  90                  95

Thr Arg Ser Pro Ser His Tyr Ser Ser Asp Pro Tyr Phe Asp Tyr Trp
            100                 105                 110

Gly Gln Gly Thr Thr Leu Thr Val Ser Ser
        115                 120

<210>  2

<211>  108
<212>  PRT
<213>  Shigella dysenteriae

<400>  2

Asp Ile Val Met Ser Gln Ser His Lys Phe Met Ser Thr Ser Val Gly
1               5                   10                  15

Asp Arg Val Ser Ile Thr Cys Lys Ala Ser Gln Asp Val Gly Thr Ala
            20                  25                  30

Val Ala Trp Tyr Gln Gln Asn Pro Gly Gln Ser Pro Lys Phe Leu Ile
        35                  40                  45

Tyr Trp Ala Ser Thr Arg His Thr Gly Val Pro Asp Arg Phe Thr Gly
    50                  55                  60

Ser Gly Ser Gly Thr Asp Phe Thr Leu Thr Ile Thr Asn Val Gln Ser
65                  70                  75                  80

Glu Asp Leu Ala Asp Tyr Phe Cys Gln Gln Tyr Ser Ser Tyr Pro Leu
                85                  90                  95

Thr Phe Gly Ala Gly Thr Ser Leu Glu Leu Lys Arg
            100                 105

<210>  3
<211>  119
<212>  PRT
<213>  Shigella dysenteriae

<400>  3

Glu Val Gln Leu Gln Gln Pro Gly Pro Glu Leu Glu Lys Pro Gly Ala
1               5                   10                  15

Ser Val Lys Leu Ser Cys Lys Ala Ser Gly Tyr Ser Phe Thr Asp Tyr
            20                  25                  30

Asn Met Asn Trp Val Lys Gln Asn Asn Gly Glu Ser Leu Glu Trp Ile
        35                  40                  45

Gly Lys Ile Asp Pro Tyr Tyr Gly Gly Pro Ser Tyr Asn Gln Lys Phe
    50                  55                  60

Lys Asp Lys Ala Thr Leu Thr Val Asp Lys Ser Ser Ser Thr Ala Tyr
65                  70                  75                  80

Met Gln Phe Lys Ser Leu Thr Ser Glu Asp Ser Ala Val Tyr Tyr Cys
                85                  90                  95

Thr Arg Gly Gly Asn Arg Asp Trp Tyr Phe Asp Val Trp Gly Ala Gly
            100                 105                 110

Thr Thr Leu Thr Val Ser Ala
        115

<210>  4
<211>  113
<212>  PRT
<213>  Shigella dysenteriae

<400>  4

Asp Ile Val Leu Ser Gln Ser Pro Ser Ser Leu Val Val Ser Val Gly
1               5                   10                  15

Glu Lys Val Thr Met Ser Cys Lys Ser Ser Gln Ser Leu Leu Tyr Ser
            20                  25                  30

Arg Asn Gln Lys Asn Tyr Leu Ala Trp Tyr Gln Gln Lys Pro Gly Gln
        35                  40                  45

Ser Pro Lys Val Leu Ile Tyr Trp Ala Ser Thr Arg Glu Ser Gly Val
    50                  55                  60

Pro Asp Arg Leu Thr Gly Ser Gly Ser Gly Thr Asp Phe Thr Leu Thr
65                  70                  75                  80

Ile Ser Ser Val Lys Ala Glu Asp Leu Ala Val Tyr Tyr Cys Gln Gln
                85                  90                  95

Tyr Tyr Ser Tyr Pro Leu Thr Phe Gly Ala Gly Thr Lys Leu Glu Leu
            100                 105                 110

Lys